# EUROPEAN PATENT APPLICATION

(11) **EP 2 819 040 A1**
(43) Date of publication of application: **31.12.2014**
(21) Application number: 13305906.3
(22) Date of filing: 27.06.2013
(51) Int. Cl.: G06F 19/00, G06Q 10/06

(54) **Sample management in laboratory environment**

(71) Applicant: bioMérieux, 69280 Marcy-L'Etoile (FR)
(72) Inventor: Termoz, Sylvain, 69001 Lyon (FR); Gorse, Jean-François, 69380 Charnay (FR); Desmolaize, Jérôme, 69380 Lozanne (FR); Deverge, Emeline, 69004 Lyon (FR)
(74) Representative: Cabinet Laurent & Charras

(57) **Abstract**

There is described herewith a sample management system for a laboratory environment. The system combines a physical support, namely a label affixed to a sample, a scanning device, and a virtual support to provide effective sample management from registration to results reading and interpretation. The label may be updated at several steps of the workflow in order to provide lab personnel with relevant dynamic data concerning past, present, and future processing steps.

## Description

### TECHNICAL FIELD

The present invention relates to the field of managing samples, analysis and data throughout the many processing steps in a laboratory environment.

### BACKGROUND OF THE ART

Some laboratories are equipped with a Laboratory Information Management System (LIMS), which involves the electronic capture and computerized manipulation of data. The LIMS may be used for simple sample tracking or as an enterprise resource planning tool that manages multiple aspects of the lab. As technologies evolve and equipment used in the lab becomes more complex and specialized, labs having a LIMS must either have a customized version that is designed to operate with its specific set of equipment, or a very simplified version that is decoupled from the equipment and has limited functionalities. On the one hand, having a customized version may be expensive and require special maintenance. On the other hand, a simplified version may not provide all of the tools that are desired by the lab personnel.

Therefore, there is a need for a laboratory management system that does not need to be customized to the equipment in the lab per se and can provide useful features to a laboratory environment.

### SUMMARY

There is described herewith a sample and analysis management system for a laboratory environment. The system combines a physical support, namely a label affixed to a sample or an analysis, and a scanning device with a virtual support to provide effective sample and analysis management from registration to results reading and interpretation. The label may be updated at several steps of the workflow in order to provide lab personnel with relevant dynamic data concerning past, present, and future processing steps.

In accordance with a first broad aspect, there is provided a laboratory sample and analysis management system comprising: a memory having stored therein a set of workflows for processing samples; a processor; and at least one application stored in the memory and executable by the processor. The application is executable for: receiving registration data for a given sample; generating a sample label comprising a first encoded symbol containing sample data and processing status for the given sample; receiving scan data from the first encoded symbol on the sample label; identifying the given sample and the processing status from the first encoded symbol; and generating an updated sample label comprising a second encoded symbol containing the sample data and an updated processing status for the given sample in accordance with the workflows.

In accordance with a second broad aspect, there is provided a computer-implemented method for managing a sample and analysis in a laboratory, the method comprising: storing a set of workflows for processing samples; receiving registration data for a given sample; generating a sample label comprising a first encoded symbol containing sample data and processing status for the given sample; receiving scan data from the first encoded symbol on the sample label; identifying the given sample and the processing status from the first encoded symbol; and generating an updated sample label comprising a second encoded symbol containing the sample data and an updated processing status for the given sample in accordance with the workflows.

In accordance with a third broad aspect, there is provided a computer readable medium having stored thereon program code executable by a processor for managing a sample and analysis in a laboratory, the program code executable for: storing a set of workflows for processing samples; receiving registration data for a given sample; generating a sample label comprising a first encoded symbol containing sample data and processing status for the given sample; receiving scan data from the first encoded symbol on the sample label; identifying the given sample and the processing status from the first encoded symbol; and generating an updated sample label comprising a second encoded symbol containing the sample data and an updated processing status for the given sample in accordance with the workflows.

In this specification, the term "multi-sample holding device" is intended to mean any apparatus used in the laboratory to hold samples or analysis, such as racks, incubators, refrigerators, cupboards, etc. The apparatus may be used to temporarily store a sample in between processing steps, or it may be used as part of sample or analysis processing, such as the incubator or a test equipment.

### BRIEF DESCRIPTION OF THE DRAWINGS

Further features and advantages of the present invention will become apparent from the following detailed description, taken in combination with the appended drawings, in which:

Fig. 1 is a flowchart of an exemplary sample processing workflow in a laboratory;

Fig. 2 is a flowchart of an exemplary embodiment for the sample registration step of figure 1;

Fig. 3a is a generic exemplary embodiment of a sample label;

Fig. 3b is an exemplary embodiment of a sample label as generated during the registration step;

Fig. 4 is a flowchart of an exemplary embodiment for the sample preparation step of figure 1;

Figs. 5a and 5b are exemplary embodiments of two updated sample labels as generated during the preparation step;

Fig. 5c is an exemplary embodiment of a rack label;

Fig. 6 is a flowchart of an exemplary embodiment for the sample inoculation step of figure 1;

Figs. 7a and 7b are exemplary embodiments of two updated sample labels as generated during the inoculation step;

Figs. 7c and 7d are exemplary embodiments of a rack labels;

Fig. 8a is a flowchart of an exemplary embodiment for the sample incubation step of figure 1;

Fig. 8b is a flowchart of an exemplary embodiment for the sample testing step of figure 1;

Fig. 9 is a schematic diagram of an exemplary sample management system;

Fig. 10 is a block diagram of an exemplary embodiment for an application of the sample management system of figure 9;

Fig. 11 is a block diagram of an exemplary embodiment for a workflow management module of figure 10;

Fig. 12 is a flowchart of an exemplary embodiment for operation of the sample management system;

Fig. 13a is a flowchart of an exemplary embodiment for operation of the sample management system during the preparation step;

Fig. 13b is a flowchart of an exemplary embodiment for operation of the sample management system during the inoculation step;

Fig. 13c is a flowchart of an exemplary embodiment for operation of the sample management system during the incubation step; and

Fig. 13d is a flowchart of an exemplary embodiment for operation of the sample management system during the testing step.

It will be noted that throughout the appended drawings, like features are identified by like reference numerals.

### DETAILED DESCRIPTION

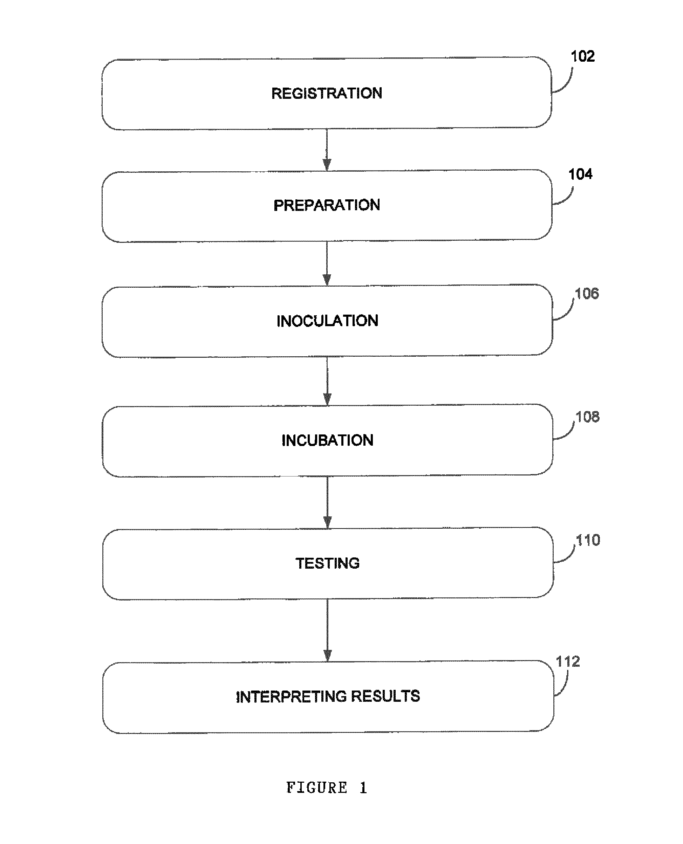

There is described herein sample and analysis management in a laboratory using a sample and analysis management system. Figure 1 is a high level flowchart illustrating the various steps of sample processing within the laboratory using the sample and analysis management system. It should be noted that the steps of figure 1 are specific to a microbiology lab and to certain types of samples but may be modified as applicable to other types of samples and analysis and/or other types of laboratories. Other steps may also be used with the sample management system, as will be understood below.

In this example, a sample will pass through six separate steps, namely registration 102, preparation 104, inoculation 106, incubation 108, testing 110, and interpreting results.

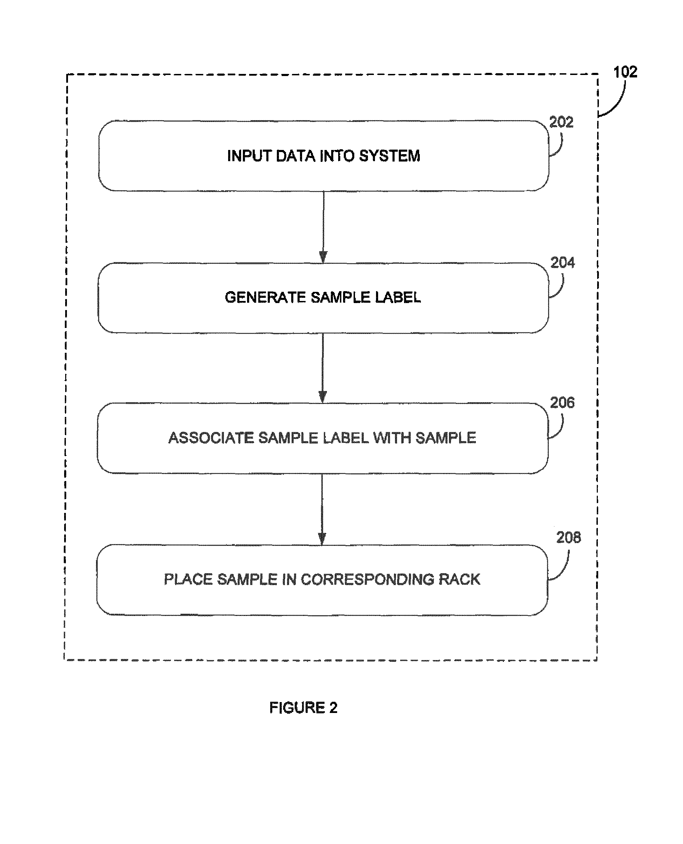

Registration 102 is the first step by which the sample is entered into the sample management system. It may be done by a client desiring to have the sample analyzed and thus performed remotely from the laboratory, or it may be done as the sample reaches the laboratory and before it begins further processing. Exemplary information provided at the time of registration 102 is sample name, sample type, origin, date of collection, location of collection, time of collection, person performing registration of sample, analysis to be performed etc. Other information helpful for the purposes of tracking, sample identification, and/or sample processing may also be provided and kept of record for each registered sample. Referring to figure 2, there is illustrated an exemplary embodiment for the step of registration 102 using the sample management system. Once data has been input into the system 202, one or more labels are generated 204 to assist in managing the sample throughout the process. The label acts as a physical support for information entered into and managed by the sample management system and helps all lab operators, whether it is a technician, an immunologist, a microbiologist, or other lab operators, in knowing the present and future actions to be taken for the sample.

At least one label is associated with the sample 206. In some embodiments, one or more labels may also be generated for the racks that will hold the sample in between the various processing steps. Associating the label with the sample/rack may comprise affixing the label with an adhesive, stapling the label to a packaging, or any other means of matching the label and the sample/rack such that they remain associated as the sample is processed. Once the label is associated with the sample, the sample is placed in a corresponding rack 208.

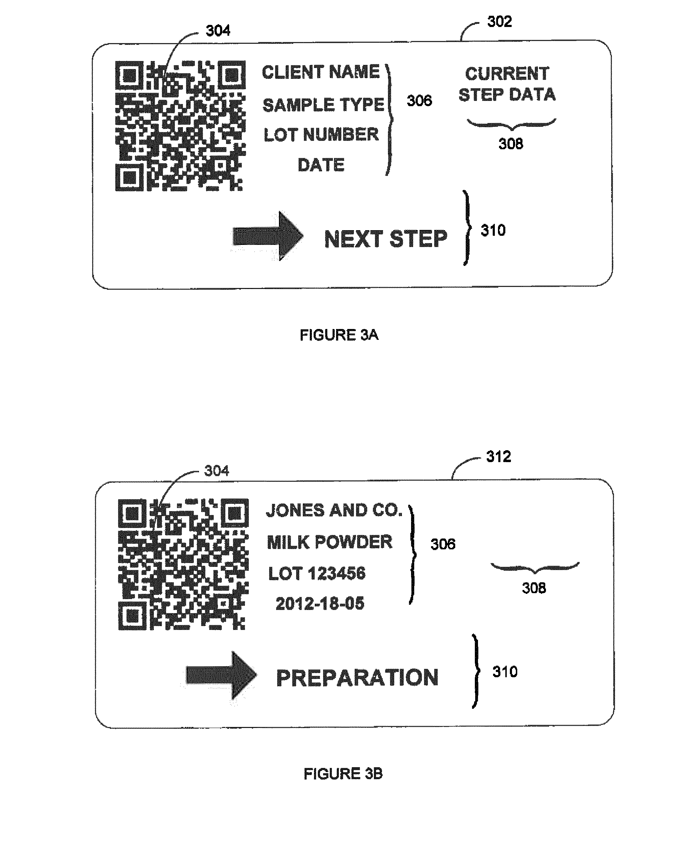

The labels may comprise data related to the sample, data related to a present step, and/or data related to a future step. Figure 3a is an exemplary generic label 302 for a sample. In this example, a 2D barcode 304 is used to store a large amount of data on a small surface. Other types of data coding, such as 1D bar codes, 2D PDF417 codes, DataMatrix codes, QR codes, MaxiCodes, and BeeTaggs may also be used to store data and allow scanning of the sample associated with the label. A scanning device may be used to read the barcode 304 and provide information to the user. Scanning the label may also provide information to the sample management system, as will be described in more detail below. For example, the code may be encoded with all relevant information needed to identify the sample and assess the status at which it presently is in its processing. The code need not be complex as the intelligence needed to identify the sample and assess its current status may be provided in the sample management system.

Sample data 306 may comprise a client name, date, a sample type, a lot number, and other identification type data for the sample. Current step data 308 may comprise information related to actions to be taken in the current step while Next step data 310 may comprise information related to actions to be taken in a subsequent step. Alternative ways of structuring the label with its different information components will be readily understood by those skilled in the art. For example, the barcode 304 may be provided along a bottom edge of the label 302 while sample data 306 is aligned on a left side, next step data is aligned on a right side, and current step data 308 is provided centrally.

Figure 3b is an exemplary sample label generated during the registration step of the process. The barcode 304 may comprise any registration data entered by a user. Scanning of the barcode by a scanning device will display this information to the user, either on a display of the scanning device or on a display of a separate device, such as a tablet, a mobile phone, or a laptop computer. The sample data 306 comprises the name of the client ("Jones and co.") as well as a sample type ("milk powder") and a lot number ("123456"). The current step data is blank as no actions need to be taken by the user during the registration step once the registration data has been entered into the system. The next step data 310 indicates that preparation is the subsequent step in the process.

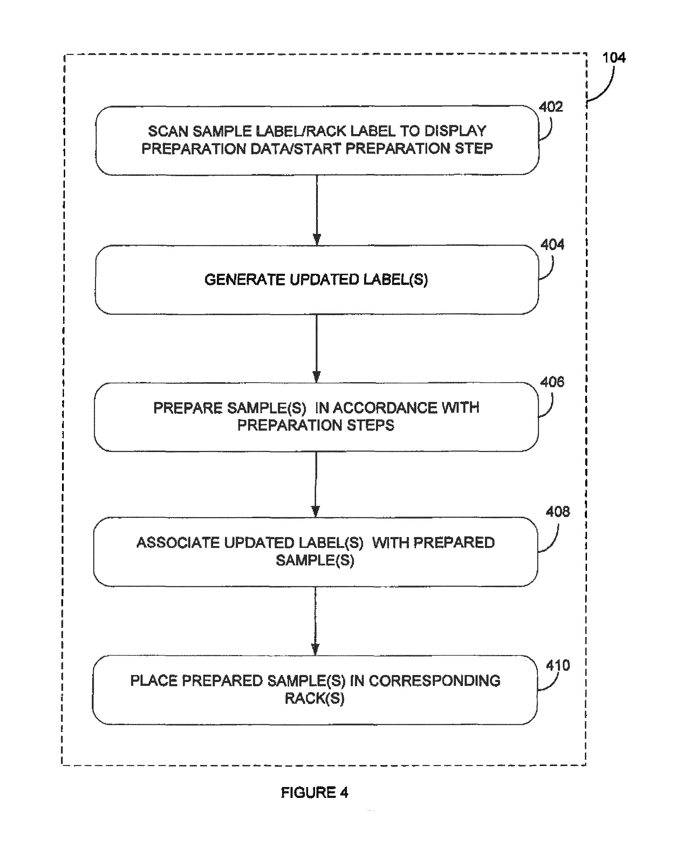

Various preparation 104 actions may be taken before the actual testing. For example, depending on the nature of the sample, it may need to be portioned into multiple particular weight or volume specimens. Each individual portion may be processed similarly or differently, depending on the nature of the testing. Other exemplary preparation steps are dilution and homogenization. Figure 4 is an exemplary embodiment for the step of sample preparation 104. In a first step 402, the sample label barcode 304 is scanned by the scanning device. This action triggers the sample management system to recognize that the preparation step has begun. Alternatively, the user may be asked to confirm that the preparation step has begun once the sample label barcode 304 has been scanned, via an interface associated with a display device. In some embodiments, the rack holding the sample may also be scanned. Scanning may also display to the user information regarding the preparation steps to be taken. The sample management system may generate a new and updated set of labels 404 at this stage. Both the updated labels and the display device interface may comprise information regarding actions to be taken during the preparation step. These actions are undertaken by the user 406 to prepare the sample and the updated labels are associated with the sample preparations 408. Updated labels may also be provided for the racks that hold the sample preparations. The prepared sample with updated labels are placed in corresponding racks 410 until the next step in the process.

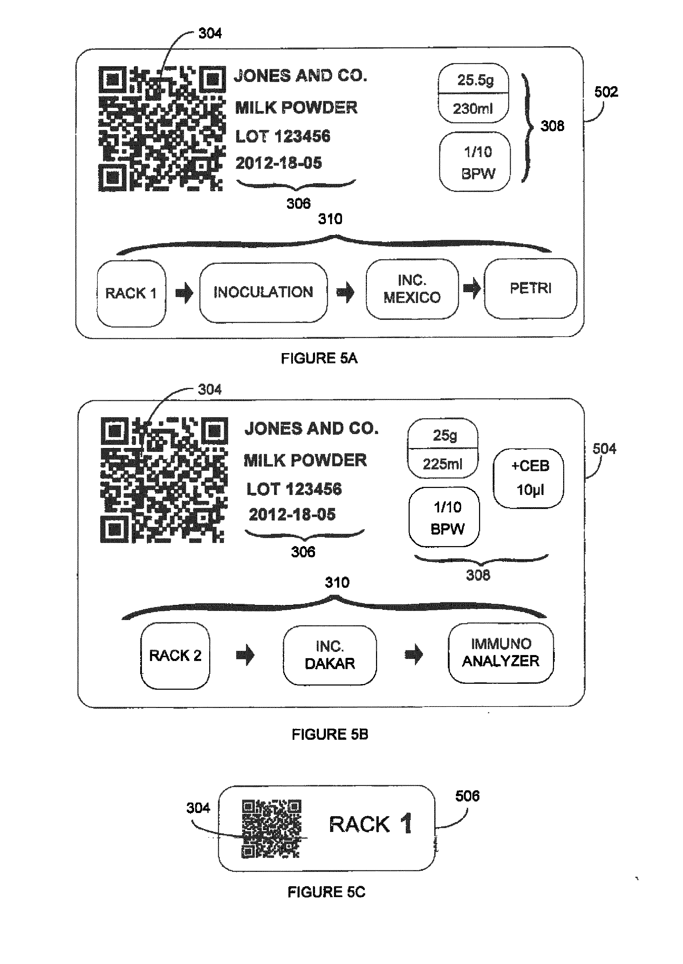

Referring now to figures 5a, 5b, and 5c, there is illustrated exemplary labels as generated by the sample management system during the preparation step. Figures 5a and 5b show updated sample labels 502 and 504, respectively, for the milk powder sample label illustrated in figure 3b. In this example, sample preparation involves separating the sample into two independent specimens, each one to be prepared and further processed in accordance with its own preparation instructions. Label 502 is associated with specimen one and label 504 is associated with specimen two. The current step data 308 indicates that 25.5g of specimen one are to be diluted into a 230ml solution, and that 25g of specimen two are to be diluted in a 225ml solution. Buffered Peptone Water (BPW) is used as a diluting agent and non-selective pre-enrichment media for detection of bacteria in a 1 to 10 ratio. In addition, specimen two is also provided with 10µl of Cronobacter Enrichment Broth (CEB), an enrichment broth for Cronobacter-type bacteria. Various symbols, acronyms, and text may be used for the current step data 308 to convey information to the lab operator handling the sample. A given lab may use its own lexicon which is known and understood by its lab personnel. Alternatively, shorthand commonly known to any lab operator may also be used. More or less detail may be provided directly on the label, as preferred and controlled via administrative settings of the sample management system.

From the next step data 310, it can be seen that specimen one will be placed in rack 1, will be inoculated, will be incubated in incubator "Mexico", and will be tested visually in a Petri dish. Meanwhile, specimen two will be placed in rack 2, incubated in incubator "Dakar", and tested in an automated immunoanalyzer. In this example, the incubators of the given lab are given names of cities around the world. Other naming schemes may be used, either more generically or more specifically, to allow the lab personnel to identify the equipment to be used for a given sample.

Figure 5c illustrates an exemplary rack label 506 for rack 1, which will hold specimen one once the preparation steps are completed before inoculation of the sample. In this example, only the name of the rack and a 2D barcode 304 are provided. The barcode 304 allows the rack scanning to form part of the workflow as it may be used to trigger a step in the process, or to enter information about the sample being processed into the sample management system. Scanning the rack also provides more precise tracking with regards to the sample and its location within the lab.

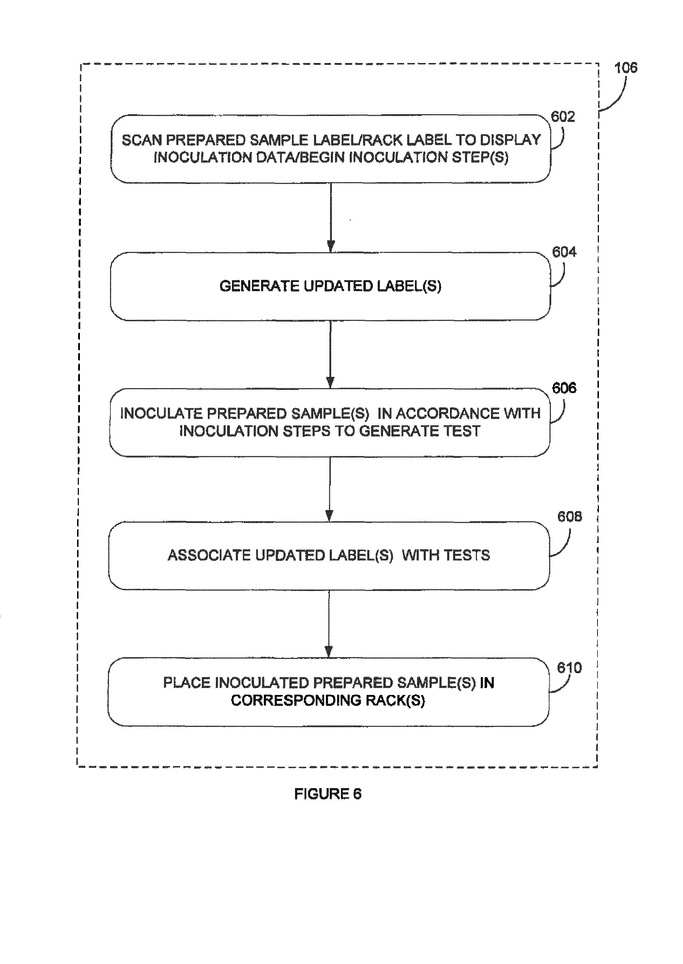

Inoculation 106 follows sample preparation 104 for some but not necessarily all types of samples, and requires that the sample, such as a microorganism or a suspension of microorganism, be introduced into a culture medium. For example, quality indicators tested for the enumeration of total flora or Enterobacteriacae are placed in conditions where the microorganisms will multiply, while samples tested for pathogens may not need to be inoculated first. The culture or growth medium may be a liquid or gel designed to support the growth of microorganisms, cells, or small plants, such as a nutrient broth or an agar plate. Figure 6 is an exemplary embodiment of the step of inoculation 106. In this example, the sample and rack labels are scanned to display inoculation data 602. Newly updated labels for the prepared sample are again generated 604 by the sample management system. Both the updated labels and the display device interface may comprise information regarding actions to be taken during the inoculation step. These actions are undertaken by the lab operator 606 by providing the sample in a culture medium and the updated labels are associated with the test 608. Similarly to the previous step, updated labels may also be provided for the racks that hold the tests. The test with updated labels are placed in corresponding racks 610 until the next step in the process.

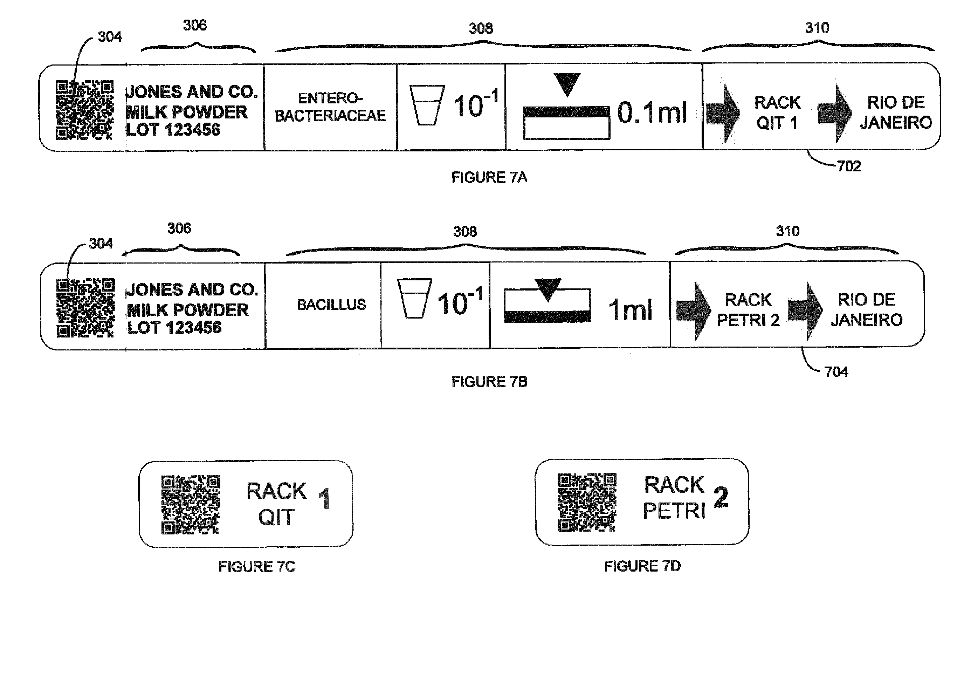

Referring to figures 7a and 7b, there is illustrated exemplary updated sample labels 702 and 704, respectively, generated during the inoculation 106 step. The current step data 308 of label 702 indicates that the dilution of sample to be used is 1/10 to enumerate "Enterobacteriaceae" in a petri plate. A 0.1ml quantity of the prepared sample is spread in a culture medium suitable for enumeration of "Enterobacteriaceae". The next step data 310 indicates that the sample will be placed in QIT rack 1 and subsequently placed in incubator "Rio de Janeiro". Similarly, label 704 indicates that the dilution of sample to be used will be 1/10 solution using a medium for Bacillus cereus enumeration. A 1ml quantity of the prepared sample is provided in a petri dish for pouring. The next step data 310 indicates that the sample will be placed in rack Petri 2 until it goes into the "Rio de Janeiro" incubator. Figures 7c and 7d are exemplary embodiments of rack labels for the QIT rack and the Petri rack. Labels may also be provided for the incubators themselves.

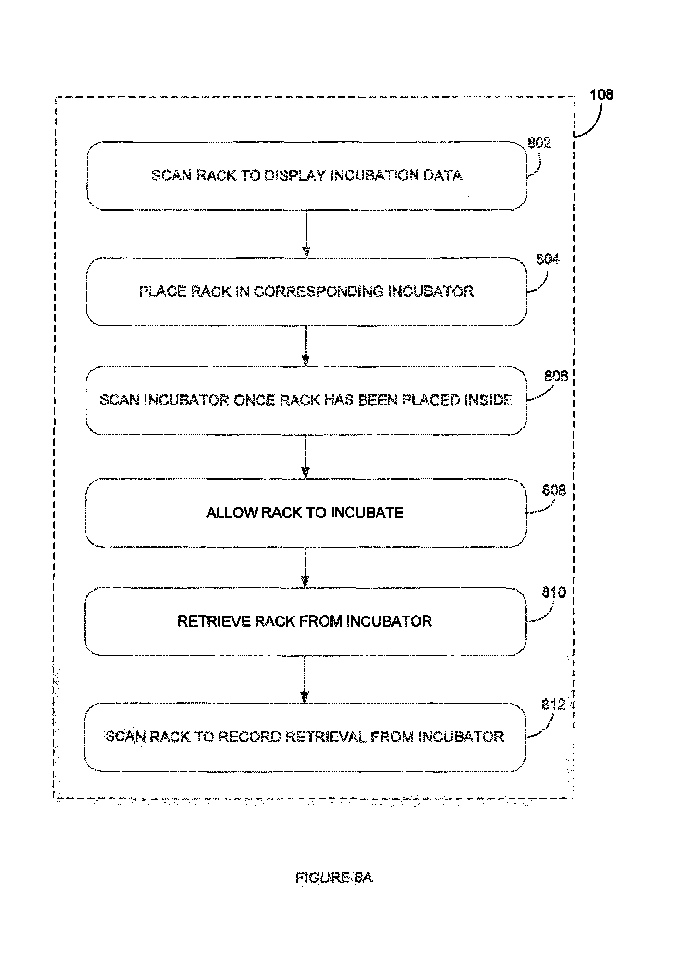

Once the inoculation is done, the sample is incubated 108 at the best temperature to grow and in the desired conditions, for example aerobic, anaerobic, etc. Figure 8a is an exemplary embodiment of the step of incubation 108. In a first step 802, the rack holding the sample is scanned. The rack is then placed in a corresponding incubator 804, as indicated on the label of the sample in the rack and/or on the display device after scanning. Once the rack has been placed inside, the incubator may be scanned 806 to indicate that incubation has begun. After the rack incubates 808, following information provided by the application, the rack is retrieved from the incubator 810 and again scanned to record the retrieval 812 in the sample management system.. Alternatively, the sample/rack is immediately taken through the steps of testing 110.

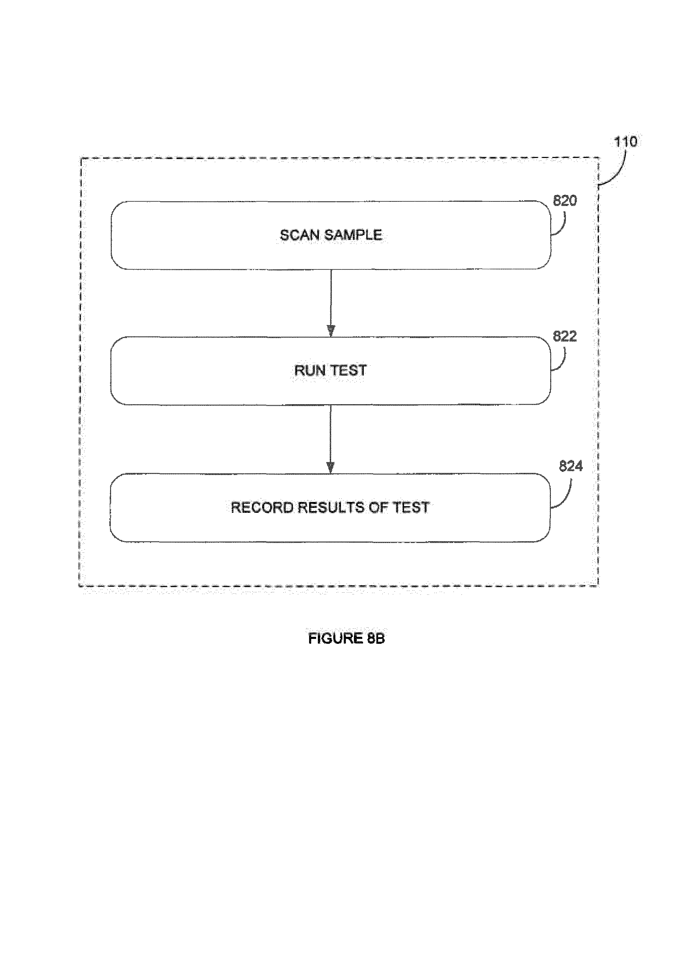

Testing 110 of the sample will follow incubation, sometimes manually and sometimes using various test equipment. Figure 8b is an exemplary embodiment of the step of testing 110. The sample may be scanned 820 and the appropriate test may be run 822. The test may be run using any known laboratory equipment, such as assay-based devices, automated quality indicators, microbial detection systems, microbial identification systems, and others. When equipment is used, the results from the test may be automatically transmitted to the sample management system for recordal 824, via a wired or wireless connection. Alternatively, results may be input manually into the system by the user. Manual tests may also be run, such as counting of Petri dish growths and others. Results are read and interpreted 112 after testing. In some embodiments, this is the last step of the process, wherein results may be consolidated/compiled in the sample management system. Recorded results may be exported and stored automatically into a memory and/or database. These results may then be accessed by a wide variety of users, as a function of access rights, such as lab personnel, clients, administrators, etc. Access may be done using any one of a wide variety of devices, such as a personal computer, a tablet, a smart phone, or the like.

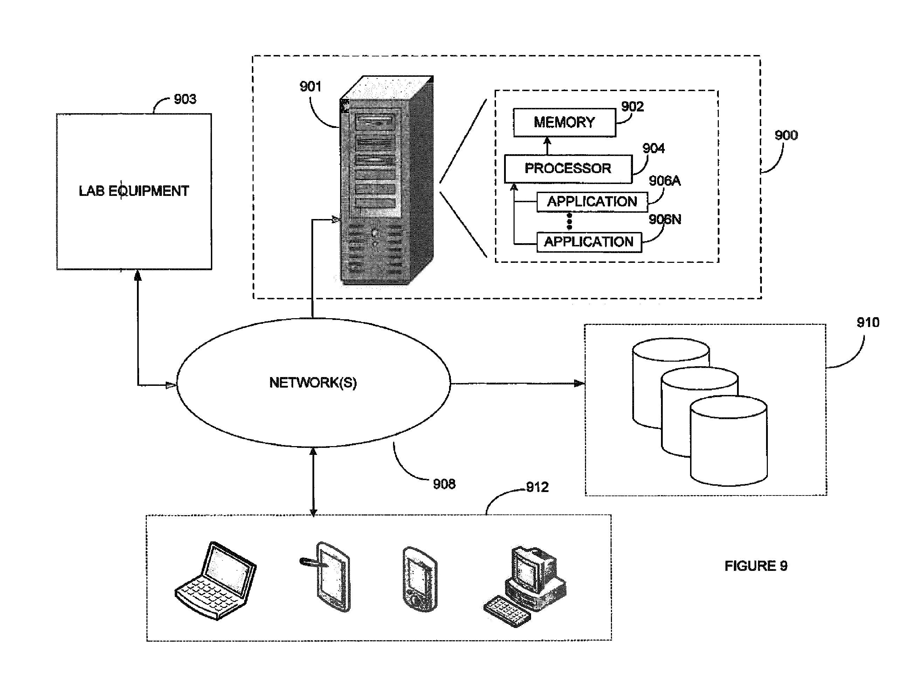

Figure 9 is a schematic diagram of an exemplary embodiment for the sample management system 900. In the embodiment illustrated, the sample management system is accessible remotely from any one of a plurality of devices 912 over a network 908. The devices 912 may comprise any device, such as a personal computer, a tablet, a smart phone, or the like, which is configured to communicate over the network 908, such as the Internet, the Public Switch Telephone Network (PSTN), a cellular network, or others known to those skilled in the art. Although illustrated as being separate and remote from the devices 912, it should be understood that the sample management system 900 may also be integrated with the devices 912, either as a downloaded software application, a firmware application, or a combination thereof. Lab equipment 903, such as incubators, analyzers, testing systems, and others, may also be accessible by the sample management system 900 via the network 908.

The sample management system 900 illustratively comprises one or more server(s) accessible via the network 908. For example, a series of servers corresponding to a web server, an application server, and a database server may be used. These servers are all represented by server 901 in Figure 9. The server 901 may be accessed by a user, such as lab personnel or a client, using one of the devices 912. The server 901 may comprise, amongst other things, a plurality of applications 906a ... 906n running on a processor 904 coupled to a memory 902. It should be understood that while the applications 906a ... 906n presented herein are illustrated and described as separate entities, they may be combined or separated in a variety of ways.

One or more databases 910 may be integrated directly into the memory 902 or may be provided separately therefrom and remotely from the server 901 (as illustrated). In the case of a remote access to the databases 910, access may occur via any type of network 908, as indicated above. The various databases 910 described herein may be provided as collections of data or information organized for rapid search and retrieval by a computer. The databases 910 may be structured to facilitate storage, retrieval, modification, and deletion of data in conjunction with various data-processing operations. The databases 910 may consist of a file or sets of files that can be broken down into records, each of which consists of one or more fields. Database information may be retrieved through queries using keywords and sorting commands, in order to rapidly search, rearrange, group, and select a field. The databases 910 may be any organization of data on a data storage medium, such as one or more servers. The databases 910 illustratively have stored therein sample data, status data, and workflow data. For example, sample data as entered by a user during the registration step may be stored therein. Actions to be taken at each step of the process may also be stored therein. Templates to generate the sample/rack labels and historical data may also be provided in the databases 910. For example, the databases 910 are secure web servers and Hypertext Transport Protocol Secure (HTTPS) capable of supporting Transport Layer Security (TLS), which is a protocol used for access to the data. Communications to and from the secure web servers may be secured using Secure Sockets Layer (SSL). Alternatively, any known communication protocols that enable devices within a computer network to exchange information may be used. Examples of protocols are as follows: IP (Internet Protocol), UDP (User Datagram Protocol), TCP (Transmission Control Protocol), DHCP (Dynamic Host Configuration Protocol), HTTP (Hypertext Transfer Protocol), FTP (File Transfer Protocol), Telnet (Telnet Remote Protocol), SSH (Secure Shell Remote Protocol)

The memory 902 accessible by the processor 904 may receive and store data. The memory 902 may be a main memory, such as a high speed Random Access Memory (RAM), or an auxiliary storage unit, such as a hard disk, a floppy disk, or a magnetic tape drive. The memory 902 may be any other type of memory, such as a Read-Only Memory (ROM), or optical storage media such as a videodisc and a compact disc. The processor 904 may access the memory 902 to retrieve data. The processor 904 may be any device that can perform operations on data. Examples are a central processing unit (CPU), a front-end processor, a microprocessor, and a network processor. The applications 906a ... 906n are coupled to the processor 904 and configured to perform various tasks as explained below in more detail. An output may be transmitted to the client device 912, as will be discussed below.

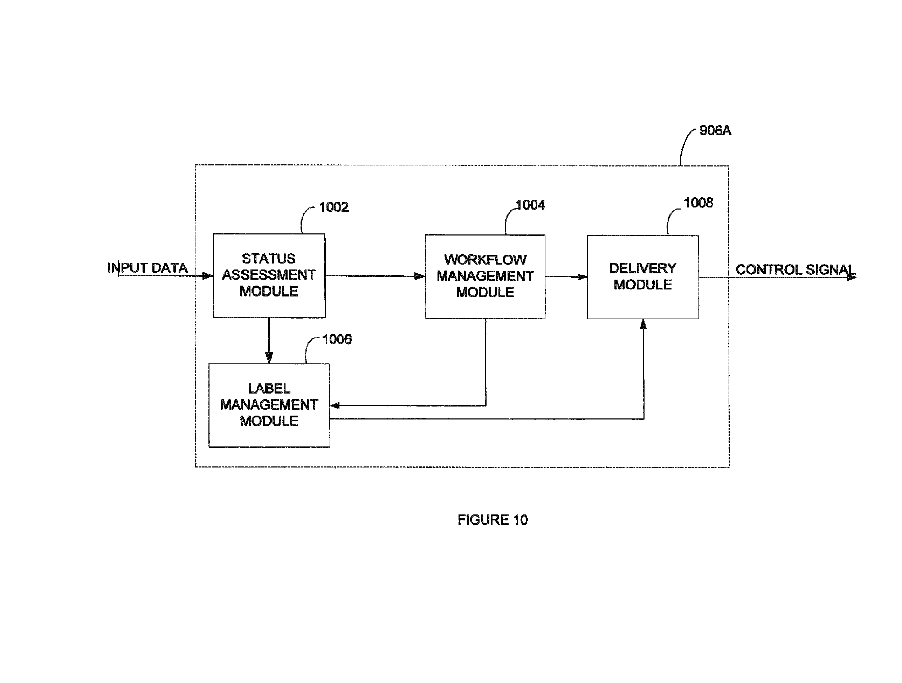

Figure 10 is an exemplary embodiment of an application 906a running on the processor 904. The application 906a illustratively comprises a status assessment module 1002, a label management module 1006, a workflow management module 1004, and a delivery module 1008. The status assessment module 1002 illustratively receives input from a scanning device, test equipment, or manually as entered by a user, such as during sample registration or testing. Such an input may comprise information about the sample, about the processing, or about the test results. For the purpose of inputting data manually, the interface presented to the user, on his/her device 912 may be used as an input means. For example, a graphical user interface comprising a plurality of user interface control elements, such as text boxes, electronic forms, drop-down boxes and menus (not shown), may be displayed on a screen of the device 912. It should however be understood that any suitable input device, such as a keyboard, a mouse, a microphone, or the like, may also be used to enter information into the system 900. Any input received from the user may be logged and stored in the memory 902 and/or databases 910 for future reference.

In order to determine how to proceed next, the status assessment module 1002 may determine what type of input has been received, which sample the input relates to, and which step of the process the input relates to. In particular and as will be discussed further below, the status assessment module 1002 may determine from the input data whether the sample is at the registration, preparation, inoculation, incubation, or testing stage. The status assessment module 1002 may also determine from the input data that a specific action, e.g. retrieval of a rack from the incubator, is currently being carried out as well as which sample the step is being carried out for.

Once the status of the sample processing has been determined, the status assessment module 1002 may send the received input data to the label management module 1006 so as to generate a label for a newly registered sample. The label management module 1006 may for this purpose store the input data in the memory 902 and/or the databases 910. In order deliver the label to a printing device, the label management module 1006 may send a control signal to the delivery module 1008. In turn, the delivery module 1008 illustratively generates a delivery control signal for causing the label to be sent to a printing device for printing. The label may also be displayed on the devices 912. In this case, the label management module 1006 may send a control signal to the delivery module 1008 to this effect. The delivery module 1008 may indeed be adapted to communicate with the devices 912 to transmit data thereto via a suitable communication means, such as email, Short Message Service (SMS), Multimedia Messaging Service (MMS), instant messaging (IM), automated voice messages, and the like. Once the data is received at a device 912, it may be retrieved and accessed by the user without having to log into the system 900. The delivery control signal may also trigger rendering of the data on the device's interface. Such an interface may comprise one or more output devices (not shown), such as a display screen, a speaker, a vibrator, or any other suitable output device.

It should be understood that the interface may simultaneously present data on multiple devices 912, and to multiple users. For example, many different lab workers may be working on different samples and/or on different steps of a same sample concurrently. The information is provided seamlessly from one device to another. In addition, a same lab worker may be consulting data on two or more devices, such as a tablet, a computer, and a mobile phone, concurrently.

The status assessment module 1002 may further send the input data and the status information to the workflow management module 1004 in order to move the sample process along. As will be discussed in further detail below, upon processing the received data, the workflow management module 1004 may then send a control signal to the delivery module 1008 for delivering data to the devices 912 using the communication means discussed above. The workflow management module 1004 may also send the control signal to the label management module 1006 to cause the latter to generate updated labels.

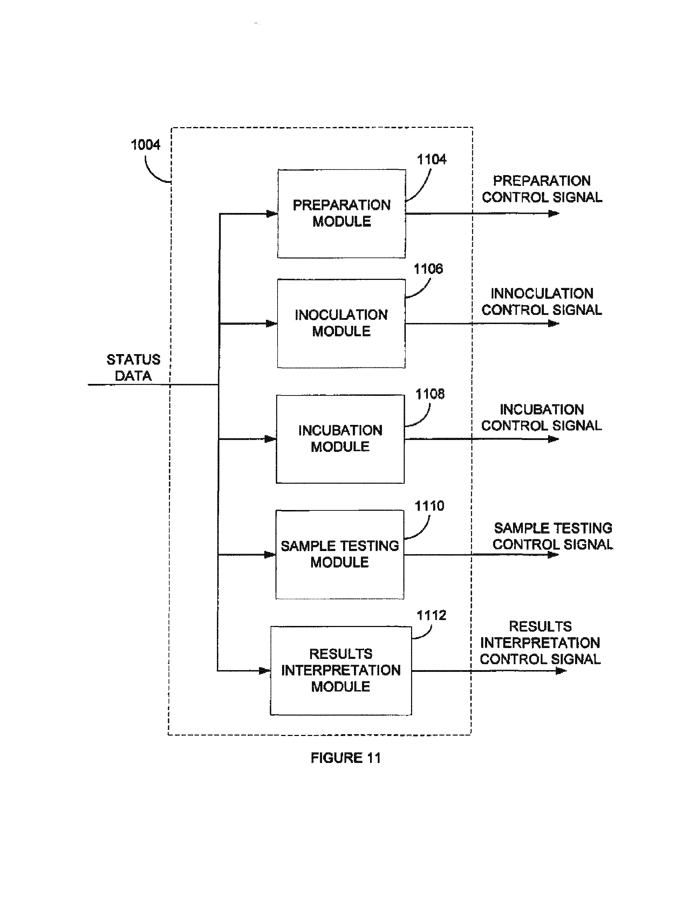

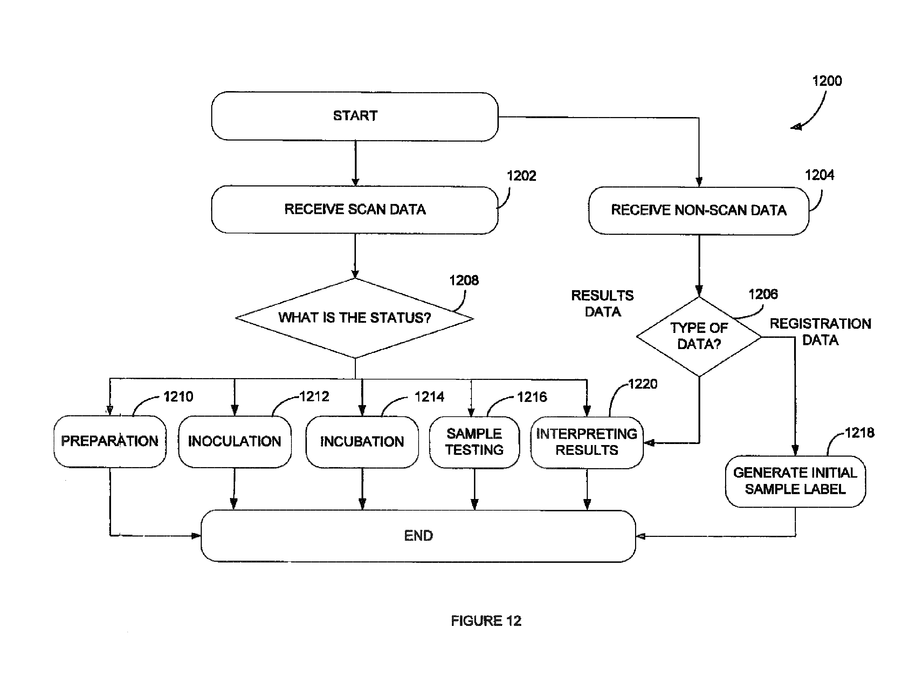

As shown in Figure 11, the workflow management module 1004 illustratively comprises a preparation module 1104, an inoculation module 1106, an incubation module 1108, a sample testing module 1110, and a results interpretation module 1112, each module managing the sample as it progresses through the process. Figure 12 is an exemplary illustration of the operation of the sample management system 900. When scan data is received 1202, the status of the sample processing is determined 1208 by the status assessment module 1002 and the system proceeds to one of preparation 1210, inoculation 1212, incubation 1214, testing 1216, or interpreting results 1220 via the workflow management module 1004. When non-scan data is received, the type of data is assessed 1206, also by the status assessment module 1002. In the case of registration data, an initial sample label is generated 1218 via the label management module 1006. If the data is results data, whether received from a testing equipment or via manual entry, the system 900 proceeds to interpreting the results 1220.

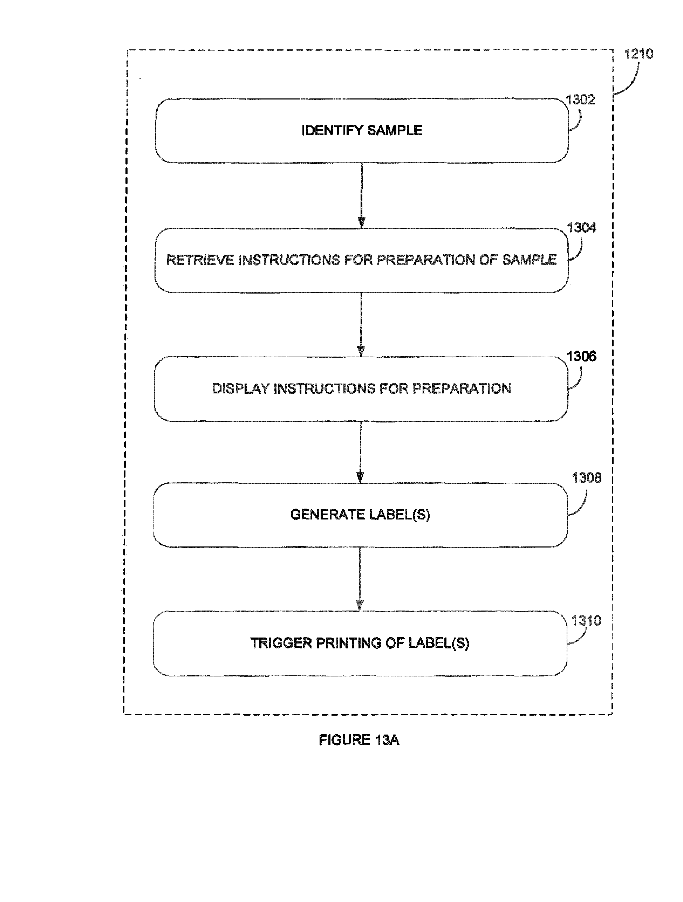

As per figure 13a, when sample management system 900 processes data through the preparation stage 1210, the sample is identified 1302 and instructions for preparation of the sample are retrieved 1304. These instructions may be retrieved from the memory 902 and/or the databases 910. The instructions for preparation may be displayed 1306 on the devices 912 and the updated labels are generated 1308 with the retrieved instructions by the label management module 1006. The delivery module 1008 will trigger printing of the updated labels 1310 at a printing device. Note that the printing device may be integrated with the scanning device.

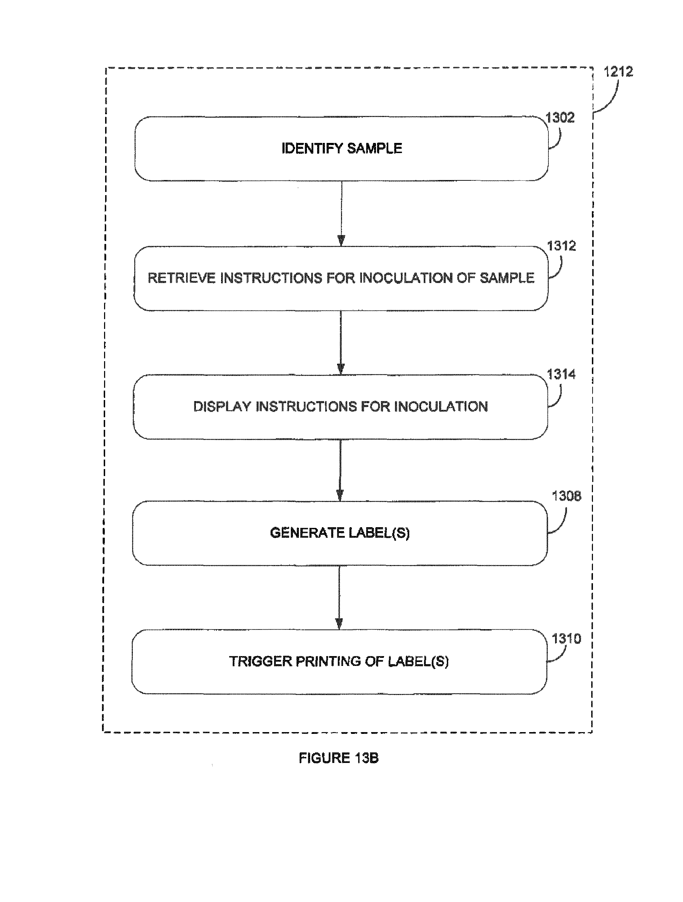

Figure 13b illustrates sample processing through the inoculation stage 1212 by the sample management system 900. The sample is again identified 1302 upon receipt of scan data and instructions for inoculation of the given sample are retrieved 1312 from the memory 902 and/or the databases 910. The instructions for preparation may be displayed 1314 on the devices 912 and the updated labels are generated 1308 with the retrieved instructions by the label management module 1006. The delivery module 1008 will trigger printing of the updated labels 1310 at a printing device.

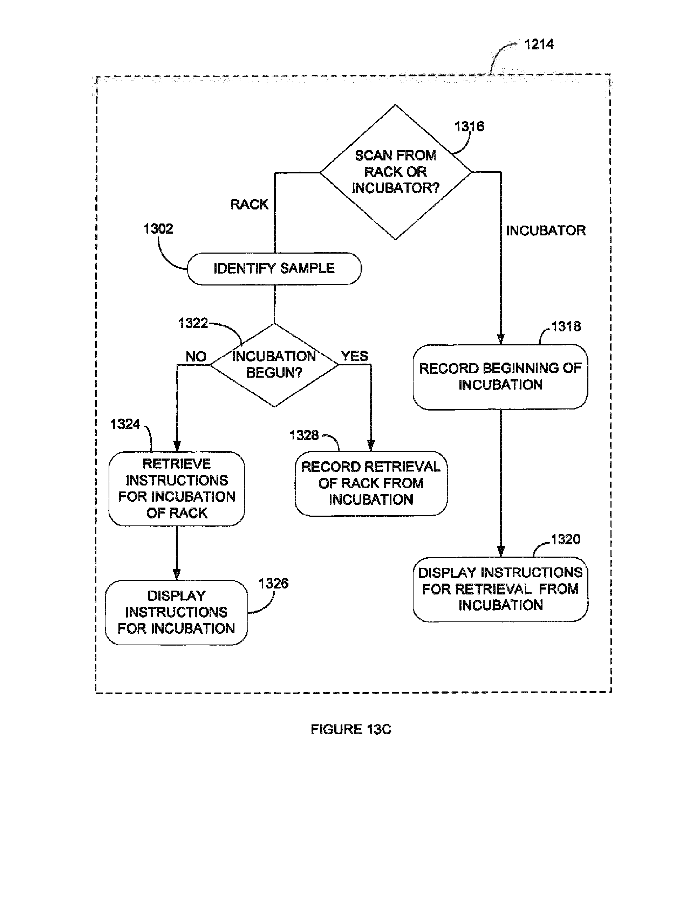

Referring now to figure 13c, there is illustrated an embodiment for sample processing through the incubation stage 1214 by the sample and analysis management system 900. The scanned data is assessed to determine if it refers to a rack or an incubator 1316. If the data comes from a rack, the sample is identified 1302 and located. In this case, it may be done indirectly by locating samples known to be in the given rack at the present time. For the samples known to be in the rack, an assessment is made as to whether incubation has begun or not 1322. If not, then instructions regarding incubation of the samples are retrieved 1324 and displayed 1326. If incubation has indeed begun, receiving scan data from the rack indicates that the samples have been retrieved from the incubator and this retrieval is recorded 1328. If the scanned data comes from an incubator label instead of a rack label, this indicates that the incubator door has been closed and incubation has begun, which is recorded 1318 by the sample management system 900. Instructions for retrieval of the samples from incubation may be displayed 1320 at the time at which incubation begins, or they may be displayed once incubation time has elapsed and the user is advised to retrieve the samples.

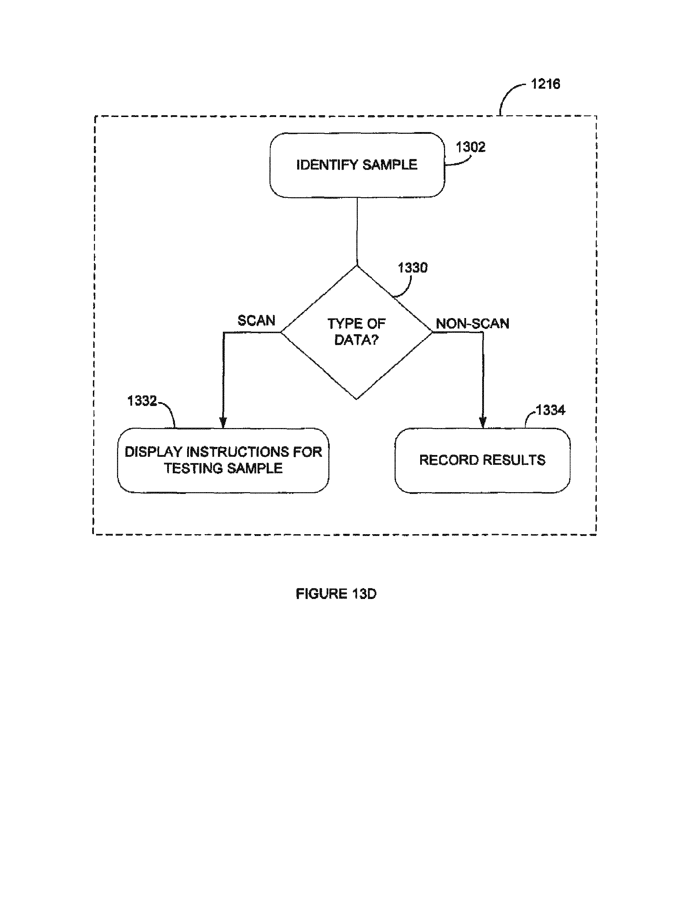

With regards to figure 13d, there is illustrated an embodiment for sample processing through the incubation stage 1216 by the sample management system 900. Receipt of data causes the sample to which the data refers to be identified 1302. Data type is assessed 1330 such that scan data triggers a display of instructions to test the sample while non-scan data causes the results of the test to be recorded.

The sample management system 900 as described above is thus adapted to provide an efficient tracking mechanism for a sample as it progresses through processing in the lab. The system 900 essentially follows the sample through the various stages of the process and keeps data entry to a minimum using the scanning device and the labels. Every step of the process may be performed by a different lab worker without any loss of information, and without the need to transfer information from one person to another. The label system allows relevant and updated information regarding the sample to be provided to the next person who will handle the sample in a simple and straightforward manner. Label generation is automated by the system 900 as the sample progresses through the processing stages and each scan of a label provides relevant information about the present actions to be taken in the context of future actions. Racks and incubators are also used as information tools, given their role in the sample management process.

While illustrated in the block diagrams as groups of discrete components communicating with each other via distinct data signal connections, it will be understood by those skilled in the art that the present embodiments are provided by a combination of hardware and software components, with some components being implemented by a given function or operation of a hardware or software system, and many of the data paths illustrated being implemented by data communication within a computer application or operating system. The structure illustrated is thus provided for efficiency of teaching the present embodiment. It should be noted that the present invention can be carried out as a method, can be embodied in a system, or on a computer readable medium. The embodiments of the invention described above are intended to be exemplary only. The scope of the invention is therefore intended to be limited solely by the scope of the appended claims.

## Claims

1. A laboratory sample management system comprising:
a memory having stored therein a set of workflows for processing samples;
a processor; and
at least one application stored in the memory and executable by the processor for:
receiving registration data for a given sample;
generating a sample label comprising a first encoded symbol containing sample data and processing status for the given sample;
receiving scan data from the first encoded symbol on the sample label;
identifying the given sample and the processing status from the first encoded symbol; and
generating an updated sample label comprising a second encoded symbol containing the sample data and an updated processing status for the given sample in accordance with the workflows.

2. The system of claim 1, wherein the at least one application is executable by the processor for retrieving instructions from the set of workflows in accordance with the processing status as identified, and providing a set of actions for advancing the given sample through its processing.

3. The system of claim 2, wherein providing a set of actions comprises at least one of displaying the set of actions on a display device and generating the updated sample label with the set of actions provided thereon.

4. The system of claims 2 or 3, wherein the set of actions comprises actions to be taken at a present time and actions to be taken at a later time.

5. The system of any one of claims 2 to 4, wherein providing a set of actions comprises providing directions for preparing the given sample for a given test.

6. The system of any one of claims 1 to 5, wherein the at least one application is executable by the processor for:
receiving scan data from the second encoded symbol on the updated sample label;
identifying the given sample and the processing status from the second encoded symbol; and
generating a further updated sample label comprising a third encoded symbol containing the sample data and a further updated processing status for the given sample.

7. The system of any one of claims 1 to 6, wherein the at least one application is executable by the processor for receiving test data related to the given sample and recording the test data.

8. The system of any one of claims 1 to 7, wherein the at least one application is executable by the processor for generating at least one holder label for at least one multi-sample holding device, the holder label comprising a fourth encoded symbol containing sample data and processing status of samples to be held by the multi-sample holding device.

9. A computer-implemented method for managing a sample in a laboratory, the method comprising:
storing a set of workflows for processing samples;
receiving registration data for a given sample;
generating a sample label comprising a first encoded symbol containing sample data and processing status for the given sample;
receiving scan data from the first encoded symbol on the sample label;
identifying the given sample and the processing status from the first encoded symbol; and
generating an updated sample label comprising a second encoded symbol containing the sample data and an updated processing status for the given sample in accordance with the workflows.

10. The method of claim 9, further comprising retrieving instructions from the set of workflows in accordance with the processing status as identified, and providing a set of actions for advancing the given sample through its processing.

11. The method of claim 10, wherein providing a set of actions comprises at least one of displaying the set of actions on a display device and generating the updated sample label with the set of actions provided thereon.

12. The method of claims 10 or 11, wherein the set of actions comprises actions to be taken at a present time and actions to be taken at a later time.

13. The method of any one of claims 10 to 12, wherein providing a set of actions comprises providing directions for preparing the given sample for a given test.

14. The method of any one of claims 9 to 13, further comprising:
receiving scan data from the second encoded symbol on the updated sample label;
identifying the given sample and the processing status from the second encoded symbol; and
generating a further updated sample label comprising a third encoded symbol containing the sample data and a further updated processing status for the given sample.

15. The method of any one of claims 9 to 14, further comprising receiving test data related to the given sample and recording the test data.

16. The method of any one of claims 9 to 15, further comprising generating at least one holder label for at least one multi-sample holding device, the holder label comprising a fourth encoded symbol containing sample data and processing status of samples to be held by the multi-sample holding device.

17. A computer readable medium having stored thereon program code executable by a processor for managing a sample in a laboratory, the program code executable for:
storing a set of workflows for processing samples;
receiving registration data for a given sample;
generating a sample label comprising a first encoded symbol containing sample data and processing status for the given sample;
receiving scan data from the first encoded symbol on the sample label;
identifying the given sample and the processing status from the first encoded symbol; and
generating an updated sample label comprising a second encoded symbol containing the sample data and an updated processing status for the given sample in accordance with the workflows.
